# EUROPÄISCHE PATENTANMELDUNG

(11) **EP 0 765 638 A1**
(43) Veröffentlichungstag der Anmeldung: **02.04.1997**
(21) Anmeldenummer: 96115396.2
(22) Anmeldetag: 25.09.1996
(51) Int. Cl.: A61B 17/36

(54) **Einrichtung zur Koagulation biologischer Gewebe mittels eines ionizierbaren Gases**

(30) Priorität: 26.09.1995 DE 19535811; 18.10.1995 DE 19538807
(71) Anmelder: Erbe Elektromedizin GmbH., D-72072 Tübingen (DE)
(72) Erfinder: Farin, Günter, 72070 Tübingen (DE); Fischer, Klaus, 72202 Nagold (DE); Schnitzler, Uwe, 72768 Reutlingen (DE)
(74) Vertreter: Bohnenberger, Johannes, Dr.

(57) **Zusammenfassung**

Die Erfindung betrifft eine Einrichtung zur Koagulation biologischer Gewebe, wobei aus Gaszuführungseinrichtungen Edelgas zu einer Gasaustrittsöffnung gefördert wird. Elektrodeneinrichtungen sind zum Leiten eines Koagulationsstroms von einer HF-Quelle in das Gas vorgesehen.

Eine derartige bekannte Einrichtung führt zu einem nicht unerheblichen Risiko für die Patienten und ist außerdem nur mit relativ großem Aufwand und schwierig handhabbar.

Es wird vorgeschlagen, Strömungseinrichtungen vorzusehen und derart auszubilden, daß das ionisierbare Gas den Raum zwischen den Elektrodeneinrichtungen und dem zu koagulierenden biologischen Gewebe im wesentlichen frei von laminaren Strömungsanteilen und im wesentlichen vollständig ausfüllt.

## Beschreibung

Die Erfindung betrifft eine Einrichtung zur Koagulation biologischer Gewebe nach dem Oberbegriff des Patentanspruches 1.

Zur Stillung von Blutungen, insbesondere bei endoskopischen Operationen, z.B. im Gastrointestinaltrakt wird u.a. die Koagulation des Gewebes durch einen HF-Koagulationsstrom angewendet. Letztendlich dreht es sich hierbei darum, daß durch den Spannungsabfall im Widerstand des stromdurchflossenen biologischen Gewebes Wärme entsteht, die das Gewebe auf eine für die Blutstillung erforderliche Temperatur erhitzt. Insbesondere dann, wenn eine großflächige Koagulation durchgeführt werden soll, besteht ein wesentliches Problem darin, daß nur an den Stellen, an denen tatsächlich eine Blutung vorliegt, eine Erwärmung des Gewebes durchgeführt und die Stellen "unbehandelt" bleiben sollen, an welchen das Gewebe intakt ist. Der Zeitfaktor spielt hier ebenfalls eine wichtige Rolle sowie auch die nicht unbeträchtliche Problematik der oftmals schwierigen Zugänglichkeit der blutenden Stelle, und zwar sowohl die Zugänglichkeit für das endoskopische Werkzeug als auch für die Beobachtung.

Aus der DE 37 10 489 C2 ist eine Einrichtung der eingangs genannten Art bekannt, die insbesondere in der offenen Chirurgie Verwendung finden kann. Ein dieser Druckschrift entnehmbarer Hauptgedanke liegt darin, daß der Gasstrom derart kräftig und (laminar) gerichtet auf das zu behandelnde Gewebe geführt wird, daß dort vorhandene Flüssigkeiten fortgeblasen werden. Dies bedeutet aber, daß der Gasstrom immer exakt dorthin gerichtet werden muß, wo zu koagulierendes Gewebe vorliegt, wobei gleichzeitig der Gasstrom zwangsläufig auch auf solche Gebiete trifft, in welchen keine Behandlung erfolgen soll. Ein weiteres, wesentliches Problem bei dieser Anordnung liegt auch darin, daß der notwendigerweise hohe Gasdurchfluß dazu führen kann, daß Gas in offenliegende Blutgefäße eintritt, was z.B. bei Verwendung von Argon als ionisierbares Gas zu erheblichen, ja sogar zu letalen Schäden führen kann.

Ein weiteres Problem bei dieser bekannten Einrichtung liegt auch darin, daß bei endoskopischen Operationen ein exaktes Richten des Gasstroms auf die zu behandelnden Flächen aufgrund der begrenzten Beweglichkeit der verwendeten Instrumente nur bedingt möglich ist.

Der Erfindung liegt die Aufgabe zugrunde, eine Einrichtung der eingangs genannten Art dahingehend weiterzubilden, daß bei verringertem Risiko für die Patienten eine erleichterte Handhabung beim Koagulieren mit geringem Aufwand erzielbar ist.

Diese Aufgabe wird durch die im Patentanspruch 1 angegebene Einrichtung gelöst.

Ein wesentlicher Punkt der Erfindung liegt darin, daß der Strom von ionisierbarem Gas, insbesondere von Argon oder Helium so geführt wird, daß dieses Gas gerade nicht die im eingangs zitierten Stand der Technik als notwendig beschriebene Wirkung aufweist, vorhandene Körperflüssigkeiten also nicht "fortbläst". Der Gasstrom ist vielmehr lediglich so stark ausgebildet, daß der relativ kleine Raum zwischen der Elektrode und dem zu koagulierenden biologischen Gewebe unter Verdrängung der Luft im wesentlichen vollständig ausgefüllt ist, ohne aber eine wesentliche mechanische Wirkung auf Flüssigkeit und Gewebe auszuüben. Dadurch ist gewährleistet, daß das zugeführte Gas nicht in offene Blutgefäße dringen und die eingangs beschriebenen Schäden verursachen kann.

Während bei der Verwendung eines laminaren Gasstroms lediglich der Bereich zwischen Auftreffstelle des Gasstroms auf das Gewebe und der Elektrode im wesentlichen frei von Umgebungsluft bleibt, wird im übrigen Raum ringsum den Gasstrahl eine relativ heftige Luftströmung aufgrund der Saugwirkung des laminaren Gasstrahls (ähnlich einer Dampfstrahlpumpe) erzielt, so daß in diesem Raum lediglich eine geringe Konzentration ionisierbaren Gases vorliegt. Durch die erfindungsgemäße Gaszuführung wird nun die gegenteilige Wirkung sichergestellt. Der gesamte, die Gasaustrittsöffnung umgebende Raum und nicht nur der Raum vor einer Düse wird mit dem zugeführten ionisierbaren Gas gefüllt, so daß sich der in einer Art "schlauchförmigen" Entladung fließende Koagulationsstrom selbstständig seinen Weg suchen kann, was nichts anderes bedeutet, als daß der Strom von der Elektrode zu den Bereichen des biologischen Gewebes fließt, welche den niedrigsten Widerstand haben. Dies sind aber gleichzeitig diejenigen Gewebebereiche (u.a. auch offene Blutgefäße), welche der Koagulationswirkung bedürften. Dies bedeutet gleichzeitig, daß der Chirurg bei einer endoskopischen Operation nicht unbedingt diejenige Stelle exakt anvisieren, d.h. sehen und auch durch bewußte Ausrichtung einer Düse treffen muß, in denen sich das zu koagulierende Gewebe befindet. Dies bedeutet nicht nur eine ganz erhebliche Erleichterung bei Operationen in schwer zugänglichen Bereichen, dies bedeutet vielmehr auch eine ganz erhebliche Beschleunigung des gesamten Koagulationsprozesses.

Vorzugsweise werden nun die Strömungseinrichtungen derart ausgebildet, daß das ionisierbare Gas aus dem biologischen Gewebe austretenden Wasserdampf langsam verdrängt und - wie oben bereits ausgeführt - nicht abrupt fortbläst. Dies führt zu einer besonders schonenden Koagulation. Aus den sich erwärmenden Gewebeabschnitten tritt nämlich bei hinreichend geringer Energiezufuhr Wasserdampf aus und bildet eine gegenüber dem ionisierten Edelgas isolierende Schicht. Dadurch kann in diese Gewebebereiche kein Koagulationsstrom mehr eintreten und die weitere Erwärmung des Gewebes wird unterbunden. Wenn nun das ionisierbare Edelgas den Wasserdampf verdrängt, beginnt der Koagulationsstrom wieder zu fließen, so daß die weitere Gewebeerwärmung erfolgt. Es liegt hier also ein im wesentlichen sich selbst regelnder Vorgang vor, der es verhindert, daß - wie es sonst manchmal passiert - das Gewebe bei zu schneller Erwärmung vom Wasserdampf zerrissen wird, der nicht schnell genug austreten kann.

Vorzugsweise sind Durchflußeinstell- oder Regeleinrichtungen vorgesehen, die derart ausgebildet sind, daß ein vorbestimmter Gas-Volumenstrom pro Zeiteinheit eingestellt werden kann und man zur Erzielung der oben beschriebenen Vorteile die Einrichtung den jeweiligen Gegebenheiten anpassen kann.

Die Strömungseinrichtungen umfassen bei einer Ausführungsform der Erfindung mindestens einen Diffusor, also eine divergierende Öffnung, durch welche sichergestellt ist, daß sich kein laminarer Gasstrom ausbilden kann, sondern eine "Wolke" von Gas aus der Öffnung ausströmt. Darüber hinaus ist es von Vorteil, wenn Verwirbelungseinrichtungen vorgesehen sind, welche dem aus der Gasaustrittsöffnung austretenden Gasstrom einen Drall verleihen oder im Inneren des Gasstroms Wirbel bilden. Dadurch wird noch sicherer gestellt, daß keine laminaren Gasströmungen auftreten können und der gesamte Raum ringsum die Gasaustrittsöffnung bis hin zur Gewebeoberfläche mit Inertgas ausgefüllt wird, ohne Gas (Luft) aus der Umgebung anzusaugen.

Weitere bevorzugte Ausführungsformen ergeben sich aus den Unteransprüchen und der nachfolgenden Beschreibung von Ausführungsbeispielen, die anhand von Abbildungen näher erläutert werden. Hierbei zeigen
- Fig. 1: eine schematisierte Darstellung einer Gesamtanordnung,
- Fig. 2: eine Düse mit Innenkontur in der bekannten Art und
- Fig. 3-7: Teilschnitte bzw. Draufsichten auf Düsen in einer Darstellung ähnlich der nach Figur 2.

In der nachfolgenden Beschreibung werden für gleiche und gleichwirkende Teile dieselben Bezugsziffern verwendet.

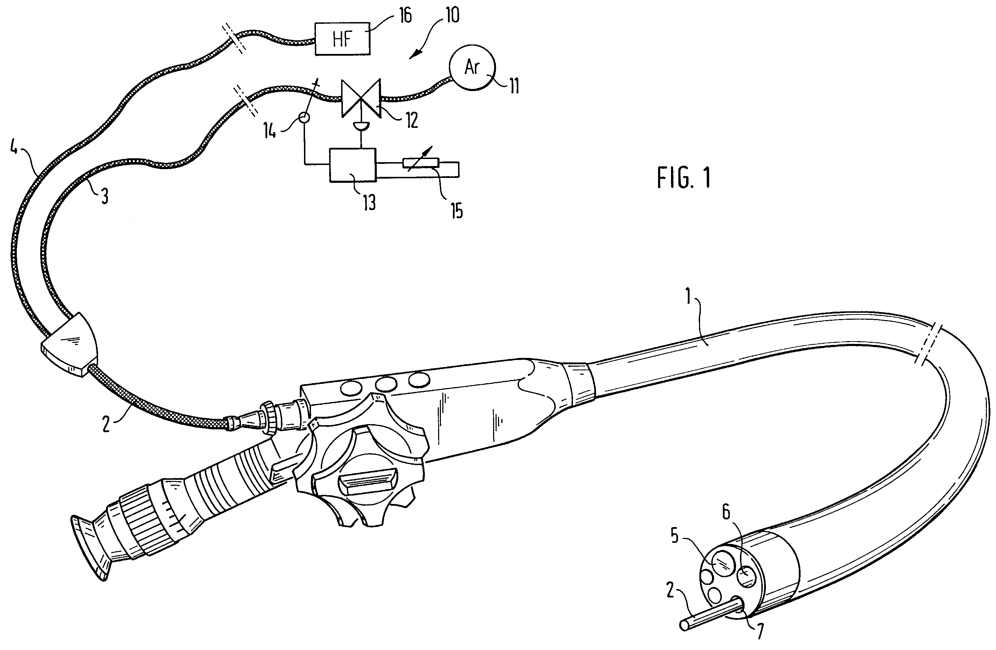

Bei der hier gezeigten, besonders bevorzugten Ausführungsform handelt es sich um eine solche, die in Zusammenhang mit einem Endoskop verwendet wird. Dieses, in Fig. 1 angedeutete flexible Endoskop 1 beinhaltet ein Rohr 2, das vorzugsweise aus PTFE ausgebildet ist und aus dem distalen Ende des Endoskops 1 herausragt. In diesem befinden sich weiterhin ein Arbeitskanal 7, in welchen das Rohr 2 geschoben ist. Im Endoskop 1 sind noch ein weiterer Arbeitskanal sowie eine Beobachtungsoptik vorgesehen. In Fig. 1 sind das Ende 6 des Arbeitskanals sowie die Linse 5 der Beobachtungsoptik gezeigt.

Das proximale Ende des Rohres 2 steht über eine Gaszuführungsleitung 3 und ein Ventil 12 mit einer Argonquelle 11, vorzugsweise einer Druckgasflasche in Verbindung. Das Ventil 12 wird über eine Stelleinrichtung in Übereinstimmung mit dem Ausgangssignal eines Reglers 13 eingestellt, das wiederum abhängig vom Ausgangssignal eines Meßorgans 14 und dessen Differenz zu einem Sollwert aus einem Sollwerteinsteller 15 gebildet wird. Das Meßorgan 14 mißt vorzugsweise den Massen- oder den Volumenstrom an Gas, das durch das Ventil 12 strömt. Diese Anordnung zum Zuführen von Argongas (oder auch eines anderen ionisierbaren Edelgases) wird in der Fig. 1 mit der Bezugsziffer 10 bezeichnet.

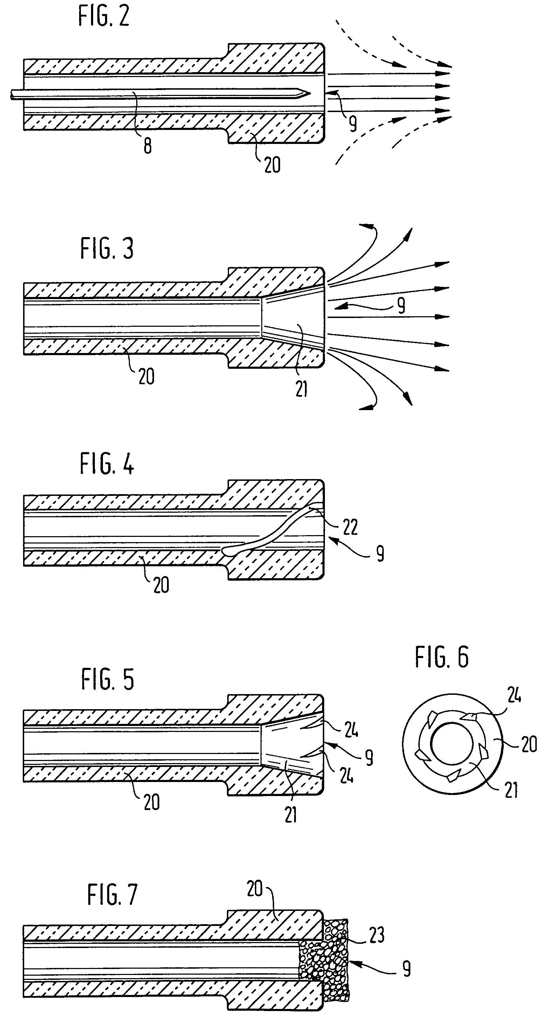

Nachfolgend werden anhand der Figuren 2 - 7 verschiedene Düsen 20 erläutert, welche (alternativ) in das distale Ende des Rohres 2 eingesetzt werden.

Die in Fig. 2 gezeigte Düse 20 ist zur besseren Erläuterung der Erfindung mit einer Innenkontur ausgestattet, wie sie an sich bekannt ist. Sie ist nämlich im Bereich der Gasaustrittsöffnung 9 zylindrisch geformt, um einen möglichst laminaren Gasstrom zu erzeugen. Im Inneren der in Fig. 2 gezeigten Ausführungsform der Düse 20 befindet sich eine Elektrodeneinrichtung 8, deren Funktion aus der eingangs genannten Druckschrift bestens bekannt ist. Der mit dieser Innenkontur erzeugte laminare Gasstrom, der in Fig. 2 mit Pfeilen angedeutet ist, hat nun die eingangs beschriebenen Nachteile. Insbesondere führt die (bei laminaren Strömungen zwangsläufig) hohe Strömungsgeschwindigkeit auch dazu, daß Luft aus der Umgebung angesaugt wird, wie dies in Fig. 2 mit unterbrochenen Pfeilen dargestellt ist.

Bei der in Fig. 3 gezeigten Ausführungsform der Düse 20 ist nun im Bereich der Gasaustrittsöffnung 9 eine Kegel- oder Diffusorbohrung 21 vorgesehen, welche nicht mehr einen laminaren Gasstrom, sondern einen divergierenden und in den Randzonen leicht verwirbelten Gasstrom erzeugt. Dadurch kommt es in den Randzonen der austretenden "Gaswolke" nur in geringem Maße zu einem Ansaugen von Umgebungsluft, so daß sich auch in den Randzonen eine hohe Konzentration an Edelgas hält. Dies wiederum führt dazu, daß sich trotz oder gerade wegen der relativ niedrigen Inertgas-Strömungsgeschwindigkeit eine "Gaswolke" zwischen der Austrittsöffnung 9 und dem (hier nicht gezeigten) Gewebe bildet, so daß sich zwischen der (auch bei den Düsen gemäß Fig. 3 - 7 natürlich vorhandenen) Elektrode 8 und dem Gewebe eine Entladungssäule bilden kann. Diese wandert nun immer zu den Stellen niedrigen Widerstands im Gewebe, also zu den Stellen, die (noch) feucht sind, so daß eine "selbsttätige" Suche nach zu koagulierendem Gewebe stattfindet. Gleichzeitig zu diesem örtlich sich selbst regelnden Vorgang findet auch eine zeitliche Regelung dadurch statt, daß der aus behandelten Gewebeabschnitten austretende Wasserdampf das Edelgas verdrängt und somit eine isolierende, unionisierte Schicht bildet. Demzufolge findet in diesen Bereichen keine (weitere) Entladung bzw. Erwärmung statt. Nach einer gewissen Zeit kondensiert der Wasserdampf bzw. wird durch das Edelgas langsam verdrängt, so daß es zu einem erneuten "Zünden" der Entladung auch in diesem Bereich kommen kann, was dann zu einer weiteren Erwärmung und Ausstoß von Wasserdampf führt, und zwar solange, bis das Gewebe soweit koaguliert ist, daß eine Entladung nicht mehr stattfinden kann bzw. der Operateur den Vorgang abbricht.

Durch diese zeitliche, selbststätige Regelung wird gewährleistet, daß ein zu schnelles Verdampfen des Wassers im Gewebe und das damit einhergehende Zerreißen von oberen Gewebeschichten zum Austritt von Wasserdampf nicht bzw. in vermindertem Maße geschieht. Die koagulierten Gewebezonen sind darum bei Verwendung der erfindungsgemäßen Einrichtung besonders schonend behandelt, so daß nur sehr kleine Poren und keine größeren Risse vorzufinden sind.

Andere Ausführungsformen solcher Strömungseinrichtungen sind in den Figuren 4 - 7 gezeigt. Bei der in Fig. 4 gezeigten Ausführungsform ist in einer an sich zylindrisch gebohrten Düse 20 eine Kerbe 22 schraubenförmig derart eingefräst, daß der ausstretende Gasstrahl einen Drall erhält, der (ebenso wie eine konische Bohrung) das Zustandekommen eines allzu scharfen, laminaren Gasstroms verhindert.

Bei der in Fig. 5 gezeigten Ausführungsform der Erfindung ist die Düse 20 am Ende nicht nur mit einer kegelförmigen Bohrung 21 ausgestattet, vielmehr sitzt in der Oberfläche des Bohrungskegels 21 auch noch eine Vielzahl von gegenüber der Längs- und der Umfangsrichtung gekippten Kerben 24, welche wiederum randseitige Verwirbelungen erzeugen und dem austretenden Gas zusätzlich noch einen Drall verleihen.

Bei der in Fig. 7 gezeigten Ausführungsform ist die Düse 20 mit einem Block aus gesinterten Kügelchen verschiedenen Durchmessers versehen, die entweder aus isolierendem Keramikmaterial aufgebaut sind (dann wird eine Elektrodeneinrichtung 8 gemäß Fig. 2 verwendet) oder aber aus leitendem Material (Metall oder Kohlenstoff oder dergleichen) bestehen und selbst als Elektrode wirken. Dann ist die Düse 20 vorzugsweise nochmals in eine isolierende Hülse derart eingesetzt, daß eine direkte Berührung mit Gewebe nicht stattfinden kann.

Der aus diesem Sinterblock 23 austretende Gasstrom ist vollständig diffus, da ein solcher Sinterblock eine Vielzahl von Öffnungen verschiedener Durchmesser und verschiedenster Formen bildet.

Die Gaszuführungseinrichtung 10 wird nun so eingestellt (durch ein entsprechendes Einstellen des Sollwerteinstellers 15), daß je nach eingesetzter Düse die oben beschriebene nicht-laminare Strömung mit Sicherheit in dem Bereich eingestellt wird, der sich zwischen der Gasaustrittsöffnung 9 und dem zu behandelnden Gewebe befindet.

Bei allen hier gezeigten Ausführungsbeispielen soll die Düse 20 aus einem hochtemperaturbeständigen Material bestehen, insbesondere aus einem Keramikmaterial, so daß der Kunststoff, aus dem das Rohr 2 gefertigt ist, in keinem Fall mit den Entladungsbereichen im Gasstrom in Berührung kommen kann. Es ist auf diese Weise auch möglich, je nach Anwendungsfall verschiedene Düsen 20 mit einem Rohr 2 bzw. eine Düse 20 mit verschiedenen Rohren 2 zu kombinieren.

### Bezugszeichenliste

- 1: Endoskop
- 2: Rohr
- 3: Gaszuführungsleitung
- 4: Verbindungsleitung
- 5: Linse
- 6: zweiter Arbeitskanal
- 7: Arbeitskanal
- 8: Elektrodeneinrichtung
- 9: Gasaustrittsöffnung
- 10: Gaszuführungseinrichtung
- 11: Argonquelle
- 12: Ventil
- 13: Regler
- 14: Meßorgan
- 15: Sollwerteinsteller
- 16: HF-Quelle
- 20: Düse
- 21: Kegel-/Diffusorbohrung
- 22: Drall-Kerbe
- 23: Sinterblock
- 24: Wirbel-Kerbe

## Patentansprüche

1. Einrichtung zur Koagulation biologischer Gewebe, umfassend
Gaszuführeinrichtungen (10) zum Zuführen eines ionisierbaren (Inert-/Edel-) Gases zu einer Gasaustrittsöffnung (9) und
Elektrodeneinrichtungen (8) zum Leiten eines Koagulationsstromes von einer HF-Quelle (16) in das Gas, **gekennzeichnet,** durch
Strömungseinrichtungen (12-14; 20), die so angeordnet und ausgebildet sind, daß das ionisierbare Gas einen Raum zwischen den Elektrodeneinrichtungen (8) und dem zu koagulierenden biologischen Gewebe im wesentlichen frei von laminaren Strömungsanteilen und im wesentlichen vollständig ausfüllt.

2. Einrichtung nach Anspruch 1,
dadurch gekennzeichnet,
daß die Strömungseinrichtungen (12-14; 20) derart ausgebildet sind, daß das ionisierbare Gas aus dem biologischen Gewebe austretenden Wasserdampf langsam verdrängt und nicht abrupt fortbläst, so daß durch die Isolationswirkung des Wasserdampfes die entsprechenden Bereiche des Gewebes von einer weiteren Einwirkung durch den Koagulationsstrom bis zur Verdrängung des Wasserdampfes geschützt sind.

3. Einrichtung nach einem der vorhergehenden Ansprüche,
dadurch gekennzeichnet,
daß die Strömungseinrichtungen Durchflußeinstell- und Regeleinrichtungen (12-14) zum Einstellen eines vorbestimmten Gas- und Volumenstroms pro Zeiteinheit umfassen.

4. Einrichtung nach einem der vorhergehenden Ansprüche,
dadurch gekennzeichnet,
daß die Strömungseinrichtungen einen in der Gasaustrittsöffnung (9) angeordneten Diffusor (21) umfassen.

5. Einrichtung nach einem der vorhergehenden Ansprüche,
dadurch gekennzeichnet,
daß die Strömungseinrichtungen eine Verwirbelungseinrichtung (24) umfassen, welche dem aus der Gasaustrittsöffnung (9) austretenden Gasstrom einen Drall verleitet und/oder Wirbel im Inneren des Gasstromes bildet.

6. Einrichtung nach einem der vorhergehenden Ansprüche,
dadurch gekennzeichnet,
daß die Strömungseinrichtungen einen Sinterblock (23) umfassen, der eine Vielzahl von vorzugsweise verschieden großen und verschieden geformten Austrittsöffnungen für das Gas bildet.

7. Einrichtung nach einem der vorhergehenden Ansprüche,
dadurch gekennzeichnet,
daß die Strömungseinrichtungen eine Düse (20) umfassen, in der sich die Gasaustrittsöffnung (9) befindet und welche in ein elastisches Rohr (2) einsetzbar ist.

8. Einrichtung nach Anspruch 7,
dadurch gekennzeichnet,
daß die Düse (20) aus hochtemperaturbestädigem Material, vorzugsweise aus Keramikmaterial besteht.
